# FASCICULE DE BREVET EUROPEEN

(11) **EP 0 698 083 B1**
(45) Date de publication et mention de la délivrance du brevet: **26.05.1999**
(21) Numéro de dépôt: 95912301.9
(22) Date de dépôt: 10.03.1995
(51) Int. Cl.: C12M 1/28, C12M 1/26

(54) **DISPOSITIF APPLICATEUR D'UN ELEMENT PLAT DE PRELEVEMENT DE MICRO-ORGANISMES, TEL QU'UNE BOITE DE PETRI**
AUFTRAGSVORRICHTUNG FÜR EIN FLACHES ELEMENT ZUR MIKROORGANISMENENTNAHME, WIE BEI EINER PETRISCHALE
APPLICATOR DEVICE FOR A FLAT MICROORGANISM COLLECTING ELEMENT SUCH AS A PETRI DISH

(30) Priorité: 10.03.1994 FR 9403027
(43) Date de publication de la demande: 28.02.1996
(73) Titulaire: BIO MERIEUX, 69280 Marcy l'Etoile (FR)
(72) Inventeur: DOLEANS, Francis, F-69009 Lyon (FR); BONNET, Alain, F-69290 Craponne (FR); BAEYAERT, Sylvain, f-69290 Saint-Genis-les-Ollières (FR)
(74) Mandataire: Guerre, Dominique
(86) Numéro de dépôt international: FR9500285
(87) Numéro de publication internationale: WO9524463

(56) Documents cités:
- EP-A- 0 150 775
- WO-A-93/17090

## Description

Le contrôle de l'hygiène est un facteur critique, notamment dans les milieux industriels et hospitaliers. A cet égard, différents systèmes ou dispositifs ont été développés pour le contrôle de l'air et des liquides. On peut citer à titre d'exemple le collecteur de germes *RCS®* de la société Biotest, pour la détermination de la teneur en germes de l'air, et l'indicateur de germes pour la détermination de la teneur en germes dans un milieu liquide, commercialisé par cette même société sous la dénomination *Hycon®.*

En dépit d'un contrôle de la qualité de l'air et des liquides, il subsiste un risque majeur lié à la contamination des surfaces. A cet égard, plusieurs sociétés ont développé des milieux de culture en boîtes appropriés qui assurent une croissance des microorganismes prélevés directement sur les surfaces contaminées, et permettent une lecture directe du nombre de colonies de la boîte. On peut citer à titre d'exemple le milieu ou boîte Count-Tact (nom commercial référence 43501), commercialisé par la société BIO MERIEUX. Les prélèvements sont effectués par application, après enlèvement du couvercle de la boîte, par pression manuelle, sur le fond de la boîte, pendant un temps non défini, de façon à ce que le milieu gélosé entre en contact le plus étroit possible avec la surface à prélever.

En dépit de l'optimisation des milieux en boites pour la croissance bactérienne après prélèvement sur des surfaces, la technique d'application manuelle utilisée jusqu'à ce jour souffre de différents inconvénients.

Un inconvénient majeur consiste en un défaut de reproductibilité des résultats, condition importante, notamment dans le suivi de la biocontamination des surfaces ou le contrôle de stérilité, principalement dû à des variations de contact entre le milieu gélosé et la surface à contrôler, en raison des différences de la force manuelle appliquée sur la boîte qui peut considérablement varier d'un opérateur à l'autre, voire avec un même opérateur lors de différents prélèvements.

Aussi a-ton été amené à concevoir un dispositif pour l'application de milieux en boîtes sur des surfaces à contrôler, qui pallie le défaut de reproductibilité des résultats, par standardisation de la pression appliquée, quelle que soit la surface à tester et son inclinaison, et qui de plus permette un contrôle de la durée d'application. Ainsi, le dispositif selon l'invention vise à standardiser le prélèvement de surface, en permettant d'obtenir une force d'appui prédéterminée dans un laps de temps contrôlé.

De manière générale, la présente invention concerne un dispositif applicateur de tout élément plat de prélèvement de microorganismes, comprenant au minimum un logement aplati pour contenir et retenir un milieu de culture réparti en couche, avec au moins un rebord disposé à l'extérieur, par exemple autour dudit logement, au-dessous de la surface libre du milieu de culture dans le logement aplati. Cette définition générale recouvre un grand nombre de réalisations différentes de l'élément plat de prélèvement, notamment celle décrite ci-après par référence aux dessins.

Pour coopérer avec un élément de prélèvement, tel que défini précédemment, le dispositif applicateur selon l'invention comprend :
- une platine,
- un piètement de support de la platine à distance de la surface contaminée à prélever ou tester, déterminant une surface de sustentation, et ménageant une ouverture pour introduire et placer sous la platine l'élément de prélèvement, dans sa position de contact avec la surface contaminée, c'est-à-dire sens dessus-dessous, parallèlement à la surface de sustentation, et avec la surface libre du milieu de culture dirigée vers le bas,
- un poussoir monté libre en translation sur la platine, perpendiculairement à la surface de sustentation, avec une tête d'appui saillant à l'intérieur du piètement,
- un moyen de rappel du poussoir vers la surface de sustentation,
- et au moins un épaulement déterminant une surface d'appui parallèle au plan de sustentation, pour retenir le rebord de l'élément de prélèvement, dans sa position de contact avec la surface à prélever ou tester, et ceci à l'encontre du poussoir.

Grâce à l'invention, il suffit de placer l'élément de prélèvement dans sa position de contact avec la surface à prélever, dans le logement intérieur déterminé par le piètement, avec une retenue du rebord dudit élément par l'épaulement dudit piètement, pour obtenir un ensemble prêt à être appliqué par l'utilisateur contre et au contact de la surface à tester. Il suffit en effet d'appliquer la surface libre du milieu de culture contre la surface à prélever, et d'appuyer contre le dispositif applicateur, ce qui a pour effet de repousser ou rétracter l'élément plat de prélèvement à l'intérieur du piètement, en amenant au moins la partie centrale de la surface libre du milieu de culture en coïncidence ou contact avec la surface à tester, et ceci à l'encontre du poussoir, et plus précisément à l'encontre de son moyen de rappel. Mais, dès lors et dans ce cas, la force d'application ou pression contre l'élément plat de prélèvement n'est plus celle appliquée par l'utilisateur, mais celle calibrée ou prédéterminée correspondant à la force de rappel exercée contre le poussoir.

La présente invention est maintenant décrite par référence au dessin annexé, dans lequel :
- la Figure 1 représente une vue de côté d'un dispositif applicateur selon l'invention, dans lequel a été engagé un élément plat de prélèvement de microorganismes,
- la Figure 2 représente une vue en coupe de l'ensemble représenté à la Figure 1, dans la position de prélèvement contre et au contact de la surface à prélever,
- la Figure 3 représente une vue de dessous du dispositif applicateur selon l'invention, représenté aux Figures 1 et 2.

Le dispositif selon l'invention est destiné à coopérer et adapté, tant en forme que dimensions, à un élément plat 1 de prélèvement de microorganismes, ayant la forme notamment d'une boite circulaire de Pétri, en matière plastique transparente. Cet élément plat, obtenu par exemple par injection ou thermoformage, comprend un logement aplati 3, contenant et retenant un milieu de culture 4, relativement solide, réparti en couche dans ledit logement, par exemple un milieu gélosé. Ce même élément plat circulaire comprend un rebord circulaire 5 disposé à l'extérieur du logement 3 au-dessous de la bordure de ce dernier, et en particulier de la surface libre 4a en forme de ménisque convexe, du milieu de culture 4. Un couvercle 17 circulaire, amovible, est associé à l'élément plat circulaire 1 de prélèvement, pour fermer par emboîtement le logement 3 du milieu de culture 4.

De manière générale, le dispositif applicateur selon l'invention, réalisé par exemple en matière plastique injectée, comprend :
- une platine 6 de forme générale triangulaire,
- un piètement 7 de support de la platine à distance de la surface contaminée à prélever, déterminant lui-même une surface de sustentation 9, et ménageant une ouverture 7a pour introduire et placer l'élément de prélèvement 1 dans une position de contact avec la surface à prélever 2, ceci parallèlement à la surface de sustentation 9, et avec la surface 4a du milieu de culture 4 dirigée vers le bas,
- un poussoir 10 monté libre en translation sur la platine 6 dans un logement 6a de cette dernière, avec une tête 11 d'appui saillant à l'intérieur du piètement, et une extrémité 21 opposée, dont la fonction sera précisée ci-après,
- un moyen de rappel ou ressort 12 du poussoir 10 vers la surface de sustentation 9,
- deux épaulements 13, situés à l'intérieur du piètement 7, et déterminant une surface d'appui 14 parallèle au plan de sustentation 9, pour retenir le rebord 5 de l'élément 1 de prélèvement, dans sa position de contact avec la surface 2 à prélever, à l'encontre du poussoir 10.

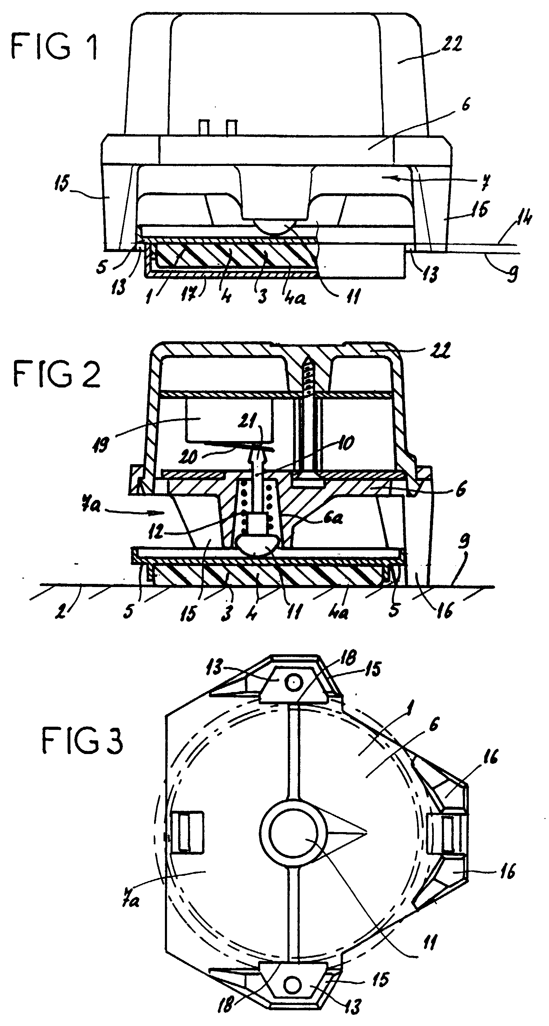

Comme montré plus particulièrement à la Figure 3, sur laquelle la position de contact avec la surface à prélever 2 de l'élément de prélèvement 1 est représentée par des traits mixtes, le piètement 7 détermine, dans le plan de la surface d'appui 14 des épaulements 13, un polygone intérieur centré sur l'élément plat 1, dans sa position de contact avec la surface à prélever 2.

L'axe du poussoir 10 passe, de manière correspondante, par le centre de ce polygone intérieur.

Toujours comme montré par la Figure 3, le piètement 7 comprend deux pieds 15 éloignés, présentant une face interne droite, séparés par une distance égale, au jeu fonctionnel près, au diamètre extérieur de l'élément 1 de prélèvement plat et circulaire, correspondant à l'ouverture 7a du piètement, ainsi que deux pieds rapprochés 16, en face de l'ouverture 7a, assurant le centrage de l'élément 1.

Fonctionnellement, la course de libre rétraction de l'élément plat 1 de prélèvement, à l'intérieur du piètement 7, à l'encontre du moyen de rappel 12, et perpendiculairement à la surface 9 de sustentation, correspond à une distance supérieure à la hauteur séparant la surface libre 4a de l'élément plat 1 du plan de sustentation 9.

Comme représenté à la Figure 1, ceci permet en particulier d'avoir une surface libre 4a du milieu de culture 4 saillant à l'extérieur du piètement 7, avant la mise au contact de l'élément 1 avec la surface à prélever 2, qui provoque la rétraction de l'élément 1 à l'intérieur du piètement 7.

Les deux épaulements 13 sont associés aux deux pieds 15 éloignés, et consistent en deux plaquettes rapportées à plat contre la face inférieure desdits pieds 15. Ces mêmes plaquettes, formant épaulements, constituent également des moyens de maintien 18 ou serrage du couvercle 17, dans la position de contact de l'élément de prélèvement 1 décrite précédemment. Ainsi l'utilisateur peut-il mettre en place l'élément plat 1 dans le dispositif applicateur selon l'invention, avec son couvercle, le retirer au dernier moment, puis le replacer sur l'élément plat, une fois le prélèvement effectué, et ceci avant l'extraction de l'élément de prélèvement du dispositif applicateur selon l'invention.

Par ailleurs, le dispositif selon l'invention comprend, dans un boîtier 22 servant à la préhension de l'utilisateur, un organe de contrôle 19 de l'application de l'élément plat 1 de prélèvement sur la surface à prélever 2, rapporté de manière amovible, mais pouvant être solidaire de la platine 6. Cet organe 19 comporte des moyens 20 d'actionnement par le poussoir 10, agencé en relation avec l'extrémité 21 de ce dernier, formant butée contre la platine 6, opposée à la tête d'appui 11 contre l'élément de prélèvement 1. L'organe de contrôle comporte de manière non représentée un moyen de temporisation, actionnant à son tour un avertisseur de la fin du temps d'application, dans la position de contact avec la surface à prélever 2, par exemple un avertisseur sonore, ou un avertisseur visuel.

Grâce à cet organe de contrôle, le dispositif applicateur selon l'invention permet, non seulement de standardiser la force d'application contre la surface à prélever, mais également le temps d'application contre cette surface.

## Revendications

1. Dispositif applicateur d'un élément plat (1) de prélèvement de microorganismes, sur une surface à prélever (2), ledit élément de prélèvement comprenant un logement aplati (3) pour contenir et retenir un milieu de culture (4) réparti en couche, avec au moins un rebord (5) disposé à l'extérieur dudit logement, au-dessous de la surface libre (4a) du milieu de culture, **caractérisé en ce que** le dispositif applicateur comprend une platine (6), un piètement (7) de support de la platine à distance de la surface (2) à prélever, déterminant une surface de sustentation (9), et ménageant une ouverture (7a) pour y engager l'élément de prélèvement (1) dans sa position de contact avec la surface (2), parallèlement à la surface de sustentation (9) et avec la surface libre (4a) du milieu de culture dirigée vers le bas, un poussoir (10) monté libre en translation sur la platine (6), perpendiculairement à la surface de sustentation (9), avec une tête (11) d'appui saillant à l'intérieur du piètement (7), un moyen de rappel (12) du poussoir vers la surface de sustentation (9), et au moins un épaulement (13), déterminant une surface d'appui (14) parallèle au plan de sustentation (9), pour retenir le rebord (5) de l'élément (1) de prélèvement, dans sa position de contact avec la surface (2) à prélever, à l'encontre du poussoir (10).

2. Dispositif applicateur selon la revendication 1, adapté à un élément plat (1) de prélèvement de forme circulaire, caractérisé en ce que, dans le plan de la surface d'appui (14) de l'épaulement, le piètement (7) détermine un polygone intérieur centré sur l'élément plat (1), dans sa position de contact avec la surface à prélever (2).

3. Dispositif selon la revendication 2, caractérisé en ce que l'axe du poussoir (10) passe par le centre du polygone intérieur.

4. Dispositif selon la revendication 2, caractérisé en ce que le piètement (7) comprend deux pieds (15) éloignés, présentant une face interne droite, séparés par une distance égale, au jeu fonctionnel près, au diamètre extérieur de l'élément plat (1) de prélèvement, et deux pieds rapprochés (16), en face de l'ouverture (7a) du piètement, assurant le centrage de l'élément de prélèvement, plat et circulaire.

5. Dispositif selon la revendication 1, caractérisé en ce que la course de libre rétraction de l'élément plat (1) de prélèvement, à l'intérieur du piètement (7), à l'encontre du moyen de rappel (12), et perpendiculairement à la surface (9) de sustentation, correspond à une distance supérieure à la hauteur séparant la surface libre (4a) de l'élément plat (1) du plan de sustentation (9).

6. Dispositif selon la revendication 1, adapté à un élément plat (1) de prélèvement avec un couvercle (17) amovible fermant le logement (3) du milieu de culture, caractérisé en ce que le piètement (7) comporte des moyens de maintien (18) du couvercle (17), dans la position de contact de l'élément plat (1) avec la surface à prélever (2).

7. Dispositif selon la revendication 6, caractérisé en ce que les moyens de maintien (18) du couvercle sont des moyens de serrage dudit couvercle.

8. Dispositif selon la revendication 1, caractérisé en ce qu'il comprend un organe de contrôle (19) de l'application de l'élément plat (1) de prélèvement sur la surface à prélever (2), rapporté ou solidaire de la platine (6), comportant des moyens (20) d'actionnement par le poussoir (10), agencés en relation avec l'extrémité (21) de ce dernier, opposée à la tête (11) d'appui contre l'élément de prélèvement (1).

9. Dispositif selon la revendication 8, caractérisé en ce que l'organe de contrôle (19) comporte un moyen de temporisation actionnant à son tour un avertisseur de fin du temps d'application, par exemple un avertisseur sonore.

10. Dispositif selon la revendication 9, caractérisé en ce que l'organe de contrôle (19) est agencé de manière amovible par rapport à la platine (6).

## Patentansprüche

1. Applikatorvorrichtung für ein flaches, zur Abnahme von Mikroorganismen von einer abzusammelnden Oberfläche (2) vorgesehenes Element (1), wobei das Abnahmeelement einen abgeflachten Aufnahmeraum (3) zum Aufnehmen und Festhalten eines schichtartig verteilten Kulturmediums (4) aufweist, mit mindestens einer außerhalb des Aufnahmeraumes unterhalb der freien Oberfläche (4a) des Kulturmediums angeordneten Umfassung (5), dadurch gekennzeichnet, daß die Applikatorvorrichtung eine Platte (6), ein Gestell (7), um die Platte im Abstand zu der abzusammelnden Oberfläche (2) zu halten, das eine Auflagefläche (9) definiert und eine Öffnung (7a) vorsieht, um darin das Abnahmeelement (1) in dessen Position für Kontakt mit der abzusammelnden Oberfläche (2) parallel zu der Auflagefläche (9) und mit nach unten weisender freier Oberfläche (4a) des Kulturmediums aufzunehmen, einen an der Platte (6) quer zu der Auflagefläche (9) frei im Sinne einer Translation montierten Stößel (10) mit einem in dem Inneren des Gestelles (7) hervorragenden Auflagekopf (11), ein Mittel zum Zurückstellen (12) des Stößels in Richtung auf die Auflagefläche (9), sowie mindestens eine eine Stützfläche (14) parallel zu der Auflagefläche (9) definierende Schulter (13) aufweist, um die Umfassung (5) des Abnahmeelementes (1) in dessen Position für Kontakt mit der abzusammelnden Oberfläche (2) gegen die Wirkung des Stößels (10) zurückzuhalten.

2. Applikatorvorrichtung nach Anspruch 1, die an ein kreisförmiges flaches Abnahmeelement (1) angepaßt ist, dadurch gekennzeichnet, daß das Gestell (7) in der Ebene der Stützfläche (14) der Schulter ein auf dem flachen Abnahmeelement (1) in dessen Position für Kontakt mit der abzusammelnden Oberfläche (2) zentriertes polygones Inneres festlegt.

3. Vorrichtung nach Anspruch 2, dadurch gekennzeichnet, daß die Achse des Stößels (10) durch die Mitte des polygonen Inneren verläuft.

4. Vorrichtung nach Anspruch 2, dadurch gekennzeichnet, daß das Gestell (7) zwei auseinanderliegende, eine gerade innere Seite aufweisende Füße (15), die durch einen Abstand getrennt sind, der abgesehen von dem funktionellen Spiel mit dem äußeren Durchmesser des flachen Abnahmeelementes (1) gleich ist, und zwei dicht beieinander liegende Füße (16) gegenüber der Öffnung (7a) des Gestelles aufweist, die die Zentrierung des flachen und runden Abnahmeelementes gewährleisten.

5. Vorrichtung nach Anspruch 1, dadurch gekennzeichnet, daß der Weg der freien Rückzugsbewegung des flachen Abnahmeelementes (1) im Inneren des Gestelles (7) gegen die Wirkung des Mittels (12) zum Zurückstellen und quer zu der Auflagefläche (9) einem Abstand entspricht, der größer ist als die Höhe, die die freie Oberfläche (4a) des flachen Elementes (1) von der Auflagefläche (9) trennt.

6. Vorrichtung nach Anspruch 1, die an ein flaches Abnahmeelement (1) mit einem abnehmbaren Deckel (17) angepaßt ist, der den Aufnahmeraum (3) des Kulturmediums verschließt, dadurch gekennzeichnet, daß das Gestell (7) Mittel zum Halten (18) des Deckels (17) in der Position für Kontakt des flachen Elementes (1) mit der abzusammelnden Oberfläche (2) umfaßt.

7. Vorrichtung nach Anspruch 6, dadurch gekennzeichnet, daß die Mittel zum Halten (18) des Deckels Mittel zum Festklemmen dieses Deckels sind.

8. Vorrichtung nach Anspruch 1, dadurch gekennzeichnet, daß sie ein Kontrollorgan (19) für die Applikation des flachen Abnahmeelementes (1) auf die abzusammelnde Oberfläche (2) aufweist, das auf die Platte (6) aufgesetzt oder mit ihr verbunden ist, und das Mittel (20) zur Betätigung durch den Stößel (10) umfaßt, die in Beziehung zu dem gegenüber dem an dem Abnahmeelement (1) liegenden Auflagekopf (11) liegenden Ende (21) des Stößels angeordnet sind.

9. Vorrichtung nach Anspruch 8, dadurch gekennzeichnet, daß das Kontrollorgan (19) ein Verzögerungsmittel umfaßt, das während seines Ablaufens einen Melder für das Ende der Applikationszeit betätigt, beispielsweise einen akustischen Melder.

10. Vorrichtung nach Anspruch 9, dadurch gekennzeichnet, daß das Kontrollorgan (19) bezogen auf die Platte (6) in abnehmbarer Weise angeordnet ist.

## Claims

1. Applicator device for a flat element (1) for sampling of microorganisms on a surface (2) from which a sample is to be taken, the said sampling element comprising a flat recess (3) for containing and retaining a layered culture medium (4), and with at least one border (5) arranged to the outside of the said recess, below the free surface (4a) of the culture medium, characterized in that the applicator device comprises a plate (6), a base (7) for holding the plate at a distance from the surface (2) from which the sample is to be taken, defining a support surface (9), and forming an opening (7a) for engaging the sampling element (1) in its position of contact with the surface (2), parallel with respect to the support surface (9), and with the free surface (4a) of the culture medium directed downwards, a pusher (10) mounted so as to be free in terms of translation on the plate (6), perpendicular to the support surface (9), with a bearing head (11) which protrudes inside the base (7), a means (12) for returning the pusher towards the support surface (9), and at least one shoulder (13) defining a bearing surface (14) parallel to the support plane (9), for retaining the border (5) of the sampling element (1) in its position of contact with the surface (2) from which the sample is to be taken, counter to the pusher (10).

2. Applicator device according to Claim 1, adapted to match a flat sampling element (1) of circular shape, characterized in that, in the plane of the bearing surface (14) of the shoulder, the base (7) defines an internal polygon centred on the flat element (1) in its position of contact with the surface (2) from which the sample is to be taken.

3. Device according to Claim 2, characterized in that the axis of the pusher (10) passes through the centre of the internal polygon.

4. Device according to Claim 2, characterized in that the base (7) comprises two feet (15) which are spaced apart, having a straight inner face and separated by a distance which, allowing for the functional clearance, is equal to the external diameter of the flat sampling element (1), as well as two feet (16) which are close to each other, opposite the opening (7a) of the base, ensuring the centring of the flat and circular sampling element.

5. Device according to Claim 1, characterized in that the course of free retraction of the flat sampling element (1) inside the base (7), counter to the return means (12), and perpendicular with respect to the support surface (9), corresponds to a distance greater than the height separating the free surface (4a) of the flat element (1) from the support plane (9).

6. Device according to Claim 1, adapted to match a flat sampling element (1) with a removable cover (17) closing the recess (3) for the culture medium, characterized in that the base (7) comprises means (18) for holding the cover (17) in the position of contact of the flat element (1) with the surface (2) from which the sample is to be taken.

7. Device according to Claim 6, characterized in that the means (18) for holding the cover are means for clamping the said cover.

8. Device according to Claim 1, characterized in that it comprises a member (19) for controlling the application of the flat sampling element (1) on the surface (2) from which the sample is to be taken, which member (19) is attached to or made integral with the plate (6), this member (19) comprising means (20) for activation by the pusher (10), these means (20) being arranged in relation with the end (21) of the pusher (10), opposite the head (11) bearing against the sampling element (1).

9. Device according to Claim 8, characterized in that the control member (19) comprises a timing means which in turn activates a device warning of the end of the application time, for example an acoustic warning device.

10. Device according to Claim 9, characterized in that the control member (19) is arranged in a removable manner with respect to the plate (6).
